# EUROPÄISCHE PATENTSCHRIFT

(11) **EP 0 733 363 B1**
(45) Veröffentlichungstag und Bekanntmachung des Hinweises auf die Patenterteilung: **05.10.2005**
(21) Anmeldenummer: 96103733.0
(22) Anmeldetag: 09.03.1996
(51) Int. Cl.: A61K 31/385, A61K 9/16

(54) **Verfahren zur Herstellung rieselfähiger R,S-Thioctsäure, R,S-Thioctsäure Granulate sowie deren Verwendung**
Process to prepare free-flowing thioctic acid, thioctic acid granulates and their use
Procédé de préparation d'acide thioctique s'écoulant librement, granulés d'acide thioctique et leur utilisation

(30) Priorität: 21.03.1995 DE 19510130
(43) Veröffentlichungstag der Anmeldung: 25.09.1996
(73) Patentinhaber: VIATRIS GmbH & Co. KG, 61352 Bad Homburg (DE)
(72) Erfinder: Bethge, Horst, 63517 Rodenbach (DE); Klostermann, Kurt, Dr., 63834 Sulsbach-Soden (DE); Möller, Roland, 63546 Hammersbach (DE); Sator, Gerhard, Dr., 64807 Dieburg (DE)

(56) Entgegenhaltungen:
- EP-A- 0 427 247
- EP-A- 0 560 092
- DE-A- 4 317 646
- DE-A- 4 338 508

## Beschreibung

Die Erfindung bezieht sich auf ein Verfahren zur Herstellung von rieselfähiger R,S-Thioctsäure, die durch Verpressung zu hochkonzentrierten festen Darreichungsformen mit Wirkstoffgehalten von mehr als 200 mg Reinsubstanz galenisch weiterverarbeitet werden kann. Insbesondere gestattet die Erfindung auch die einfache Darstellung von hochdosierten Tabletten mit 600 mg oder mehr Reinwirkstoffgehalt.

R,S-Thioctsäure (D,L-alpha-Liponsäure) wird in pharmazeutischen Formulierungen sowohl in Infusionslösungen als auch als feste galenische Formulierung zur oralen Anwendung eingesetzt. Wenn im folgenden von Thioctsäure die Rede ist, sind immer sowohl die enantiomerenreinen Verbindungen (R- oder S-Thioctsäure) als auch das racemische Gemisch (R,S-Thioctsäure) und Mischungen mit beliebigen Gehalten an Enantiomeren zu verstehen.

Für die pharmazeutische Anwendung wird i. d. R. auf synthetischem Weg gewonnene D,L-Thioctsäure, die über die Vorstufe der Dihydroliponsäure (6,8-Dimercaptooctansäure) durch Oxidation gewonnen wird, eingesetzt. Das synthetische Verfahren kann hier auch so durchgeführt werden, daß aus enantiomerenreiner R-oder S-Dihydroliponsäure enantiomerenreine R- oder S-Thioctsäure (D- oder L-alpha-Liponsäure) erzeugt wird (DE 41 37 773, EP 0 427 247). Enantiomerenreine R-Thioctsäure oder S-Thioctsäure ist auch nach dem in der DE-OS 36 29 116 beschriebenen Verfahren zugänglich und besonders vorteilhaft einsetzbar.

Literaturbekannte Verfahren zur Herstellung von Thioctsäure-Formen umfassen u. a. Verfahren (Chem. Der. 1959, 1177), bei denen die Dimercaptooctansäure destilliert und nach Oxidation zur Thioctsäure erneut durch Destillation gereinigt wird, um dann schließlich bei -70 °C aus Essigester kristallin erhalten zu werden.

Eine weitere Methode (J. Am. Chem. Soc. 77 (1955), 416) verwendet das nach der Oxidation von Dimercaptooctansäure durch Einengen der organischen Lösungsmittel erhältliche viskose Öl. Dieses wird mit Skellysolve B mehrfach extrahiert, wobei ein wechselnder Anteil eines "polymeren" Materials zurückbleibt. Die vereinigten Extrakte werden angeimpft und bei Raumtemperatur oder während einiger Stunden im Kühlschrank kristallisiert. Die Umkristallisation aus Skellysolve B ergibt schließlich analysenreines Produkt mit einem Schmelzpunkt von 61 °C - 62 °C.

Eine weitere Vorschrift (J. Am. Chem. Soc. 77 (1955) 5148) empfiehlt zur Extraktion und Kristallisation Cyclohexan wobei auch enantiomerenreine (+)-alpha-Liponsäure auf analogem Wege erhalten wurde (J. Chem. Soc. Perkin Trans. 1 (1990), 1615).

Technische Verwendung findet heute ein Verfahren (DE 42 35 912), das organische Lösungsmittel mit einer Dielektrizitätskonstante ε von 2,5 bis 5,5 für die Extraktion und Kristallisation vorsieht.

Die bisher am Markt erhältliche Ware liegt jedoch in einer für die galenische Weiterverarbeitung zu festen Darreichungsformen nicht sehr günstigen Form vor (mangelnde Rieselfähigkeit, ungünstige Korngrößenverteilung).

Insbesondere bei der Herstellung von hochkonzentrierten Tabletten durch Verpressung, zeigen die bisher erhältlichen Thioctsäure-Formen ausgesprochen schlechte Eigenschaften. Diesem Problem konnte bislang nur durch Abmischen verschiedener, am Markt erhältlicher Thioctsäure-Formen zwecks Einstellen einer bestimmten Korngrößenverteilung und anschließender intensiver Granulation begegnet werden.

So ist es praktischer Stand der Technik, eine Mischung aus ca. 40 Gew.-Teilen Thioctsäure, die durch Umkristallisation beispielsweise gemäß der DE-OS 42 35 912 gewonnen wurde, mit 60 Gew.-Teilen Thioctsäure, die vermutlich durch ein Destillations- und/oder Schmelzverfahren aufgereinigt und durch Brechen der Schmelze erhalten wurde, herzustellen und diese Mischung unter Zusatz von ca. 15 Gew. % Hilfsstoffen, bezogen auf 100 Gew. % Thioctsäure, zu entsprechenden festen Darreichungsformen zu verpressen. Dabei ist es auch bekannt, die 60 : 40 Mischung in einem Wirbelbett einer geeigneten Vorrichtung vorzulegen, die Hilfsstoffe aus wässriger Lösung auf die Vorlage aufzusprühen, und das erhaltene Produkt zu einem verpreßbaren Granulat zu mahlen.

Dieses in der Praxis heute angewendete Verfahren ist gleich auf mehrfache Weise besonders nachteilig. Das Abmischen "verschiedener" am Markt erhältlicher Thioctsäure-Chargen führt nicht in beliebigen sondern nur in ganz bestimmten Kombinationen, beispielsweise der oben genannten, zum erwünschten Erfolg. Darüber hinaus bereitet selbst eine "erprobte" Mischung von Thioctsäuren unterschiedlichen Ursprungs häufig in der Hinsicht Probleme, daß hochdosierte feste Darreichungsformen, also z. B. Tabletten mit 600 mg Thioctsäuregehalt oder darüber, selbst bei Einhaltung der bekannten Anforderungen nicht in der erforderlichen Qualität herstellbar sind. Erschwerend kommt hinzu, daß man zwar bislang durchaus weiß, daß das Korngrößenspektrum der Thioctsäuremischung eine sehr wichtige Größe für die Verpressung darstellt, daß man aber andererseits bislang nicht in der Lage ist, die Anforderungen hinsichtlich des Korngrößenspektrums für eine Verpressung exakt zu spezifizieren. Ein Aspekt, der die Verpreßbarkeit der Thioctsäure zu Tabletten positiv beeinflußt, ist der Zusatz von basischen Substanzen, wie in DE 4 317 646 beschrieben.

Angesichts des angegebenen und diskutierten Standes der Technik war es mithin Aufgabe der vorliegenden Erfindung ein Verfahren der eingangs erwähnten Gattung anzugeben, das die Herstellung einer reinen, pharmazeutisch einsetzbaren Thioctsäure-Form gestattet, die eine leichtere galenische Weiterverarbeitung für feste Darreichungsformen gewährleistet und die insbesondere für die Verpressung zu hochkonzentrierten Darreichungsformen, ohne zwangsweise Abmischung mit anderen erhältlichen Thioctsäure-Formen, geeignet ist.

Aufgabe der Erfindung war auch die Angabe einer neuen Thioctsäure-Form mit Verarbeitungseigenschaften, welche die bekannten Thioctsäuren zumindest hinsichtlich der Eignung für hochdosierte Darreichungsformen übertrifft. Gleichzeitig sollte eine Ware mit vergleichsweise niedrigen Restlösungsmittel-Gehalten (verglichen mit am Markt erhältlichen Thioctsäure-Formen) herstellbar sein.

Die der Erfindung zugrundeliegende Aufgabe sowie weitere nicht näher bezeichnete Probleme werden durch ein Verfahren der eingangs erwähnten Art mit den Merkmalen des kennzeichnenden Teils von Anspruch 1 gelöst. vorteilhafte Verfahrensabwandlungen werden in den auf Anspruch 1 rückbezogenen Ansprüchen angegeben. in produkttechnischer Hinsicht stellt der Gegenstand von Anspruch 15 eine Lösung der Aufgabe der Erfindung dar, während bevorzugte Ausführungsformen in den abhängigen Stoffansprüchen unter Schutz gestellt werden.

Dadurch, daß Thioctsäure beliebigen Ursprungs im Wirbelbett einer zur Erzeugung einer Wirbelschicht geeigneten Vorrichtung vorgelegt wird und daß durch Versprühen einer Lösung von Thioctsäure beliebigen Ursprungs auf die Vorlage unter gleichzeitiger Entfernung des Lösungsmittels ein aus Thioctsäure bestehendes Aufbaugranulat erhalten wird, gelingt es, eine Thioctsäure-Form bereitzustellen, die
1. rieselfähig ist;
2. mit einem Einsatz von weniger als 20 Gew. % Hilfsstoffen für die galenische Weiterverarbeitung verarbeitbar ist;
3. ohne, daß die Notwendigkeit bestünde, durch Mischen zweier Thioctsäurechargen unterschiedlicher Herkunft, eine verarbeitbare Mischung einzustellen, weiterverarbeitet werden kann; und
4. dabei hochkonzentrierte feste Darreichungsformen mit Wirkstoffgehalten von mehr als 200 mg Reinsubstanz erhältlich macht, vor allem durch Verpressung zu Tabletten.
5. Darüber hinaus zeigt die erfindungsgemäße Ware deutlich niedrigere Restlösungsmittelgehalte als bislang bekannte Thioctsäure-Formen.

Im Rahmen der Erfindung wird dabei unter R,S-Thioctsäure beliebigen Ursprungs oder beliebiger Herkunft eine Ware verstanden, die hinsichtlich ihrer Reinheit grundsätzlich den aus der Verwendung als pharmazeutisches Produkt resultierenden Anforderungen genügt. Die einsetzbaren R,S-Thioctsäuren sind demnach keine Rohprodukte, können jedoch eine gewisse Menge an Verunreinigungen aufweisen, die beispielsweise aus einem Reinigungsprozeß, wie etwa der Umkristallisation aus einem Lösungsmittel, stammen.

Voraussetzung für die Eignung der Thioctsäure für das erfindungsgemäße Verfahren ist somit nur die Flüchtigkeit der ggf. in der Thioctsäure enthaltenen Verunreinigungen unter den für die Durchführung des erfindungsgemäßen Herstellungsverfahrens geeigneten Bedingungen von Druck und Temperatur.

Zu den gemäß der Erfindung mit Vorteil als Vorlage einzusetzenden Thioctsäuren gehören daher u. a. die gemäß den Verfahren aus der DE-A 42 35 912, J. Am. Chem. Soc. 77 (1955), 5148, J. Chem. Soc. Perkin Trans. 1 (1990), 1615, J. Am. Chem. Soc. 77 (1955), 426, Chem. Ber. 1959, 1177, DE-A 36 29 116, EP-A 0 427 247, DE-A 41 37 773 erhältlichen sowie die nach dem hierin beschriebenen Verfahren selbst zugängliche R,S-Thioctsäure-Form.

Die Möglichkeit, hochkonzentrierte feste Darreichungsformen aus der nach dem erfindungsgemäßen Verfahren zugänglichen Ware herzustellen, ist von besonderem Vorteil. Unter hochkonzentriert wird in der Erfindung eine Darreichungsform verstanden, deren Wirkstoffanteil > 80 Gew. % liegt. D. h., dadurch, daß der Anteil galenischer Hilfsstoffe < 20 Gew. % gehalten werden kann, sind hochdosierte Darreichungsformen, beispielsweise Tabletten, besser applizierbar.

Übliche Hilfsstoffe sind dem Galeniker geläufig.

Die Thioctsäure wird zur Erzielung eines erfindungsgemäßen Produkts im Wirbelbett einer zur Erzeugung einer Wirbelschicht geeigneten Vorrichtung vorgelegt, wobei es sich versteht, daß die Thioctsäure-Vorlage bei Ausführung der Erfindung selbst die Wirbelschicht ausbildet. Hierbei bedeutet der Begriff "Wirbelschicht" im Zusammenhang mit der Erfindung, daß die Vorlage beispielsweise als feinkörniges Schüttgut auf perforierten Böden lagernd von unten von Gasen durchströmt wird, wobei sich unter bestimmten Strömungsbedingungen ein Zustand einstellt, der dem einer kochenden Flüssigkeit ähnelt. Die zur Erzeugung einer Wirbelschicht geeigneten Vorrichtungen sind dem Fachmann bekannt und z. B. bei Winnacker-Küchler (3.) 7: 31 - 63, Mathur et al. (Adr. Chem. Engineer. 9 (1974)), Baerns (Chem. Ing. Techn. 40 (1968) 737 - 39) oder Simon (Chemie-Techn. 5 (1976) 277 - 80) beschrieben.

Auf die Thioctsäure-Vorlage im Wirbelbett, also letztlich auf die Thioctsäure-Wirbelschicht wird nun erfindungsgemäß eine Lösung von Thioctsäure beliebigen Ursprungs aufgesprüht. Das Aufsprühen oder Versprühen der Thioctsäurelösung geschieht dabei im Sinne einer Sprühtrocknung, wobei also gleichzeitig das Lösungsmittel entfernt und die Thioctsäure auf die Vorlage ähnlich einer Aufbaugranulation oder ähnlich eines Wirbelschichtcoatingverfahrens aufgebracht wird.

Grundsätzlich kann man beim Verfahren der Erfindung als stationäre Phase, d. h. als Produktvorlage in der Wirbelschicht trockene oder wahlweise auch lösungsmittelfeuchte Thioctsäure einsetzen. Dabei ist die Verwendung lösungsmittelfeuchter Thioctsäure besonders vorteilhaft, da auf diese Weise ein zusätzlicher Trocknungsvorgang eingespart werden kann.

Die Menge an stationärer Phase im Verhältnis zur Menge der aufgesprühten Phase an Thioctsäure ist erfindungsgemäß nicht besonders kritisch. Allerdings sollte die Menge der stationären Phase, bezogen auf die Gesamtmenge an erhaltenem Produkt, einen Anteil von ca. 10 Gew. % nicht unterschreiten, da ansonsten der Erfolg des erfindungsgemäßen Verfahrens in Frage gestellt sein könnte. Ferner ist es nicht besonders sinnvoll, größere Mengen als 50 Gew. % an stationärer Phase in der Wirbelschicht vorzulegen, da mit Ausnahme desjenigen Falles, in dem als stationäre Phase bereits ein aus dem erfindungsgemäßen Verfahren resultierendes Produkt eingesetzt wird, der Anteil der Beschichtung des Produkt u. U. nicht mehr ausreichen kann, um die vorteilhaften Eigenschaften des erfindungsgemäßen Produktes über das vollständige Eigenschaftsspektrum zu gewährleisten. Unter praktischen Gesichtspunkten hat es sich als besonders vorteilhaft erwiesen, wenn ca. 25 Gew.-% stationäre Phase einer Thioctsäure beliebigen Ursprungs vorgelegt werden, auf welche die restlichen 75 Gew. % aus der Lösungsmittel-Phase aufgesprüht werden. Dabei beziehen sich die Gew. %-Angaben hinsichtlich der Thioctsäure immer auf 100 Gew. % trockene Thioctsäure, wobei diese trockene Thioctsäure das Produkt meint, welches die Summe aus vorgelegter Phase und aufgesprühter Thioctsäure am Ende der erfindungsgemäßen Aufbaugranulation umfaßt.

Wie bereits weiter oben angedeutet, ist der Ursprung der als Vorlage bzw. als Wirbelschicht im Wirbelbett vorgelegten Thioctsäure beliebig. Dennoch hat sich gemäß der Erfindung in einer besonders bevorzugten Verfahrensvariante der Einsatz lösungsmittelfeuchter Thioctsäure als äußerst günstig erwiesen, welche einem Herstellungsverfahren entstammt, bei dem man ein Teil Thioctsäure bei 10 °C bis 60 °C in 5 - 20 Teilen Lösungsmittel oder Lösungsmittelgemisch löst und innerhalb von 2 bis 10 Stunden auf 0 °C bis -20 °C abkühlt, wobei das Lösungsmittel oder Lösungsmittelgemisch eine Dielektrizitätskonstante ε zwischen 2,5 und 5,5 aufweist. Diese Herstellungsvariante entspricht dem in der DE-A 42 35 912 offenbarten Verfahren, wobei der Einsatz der nach diesem Verfahren erhältlichen Thioctsäureware im erfindungsgemäßen Verfahren ein ganz ausgezeichnet zu hochkonzentrierten Darreichungsformen in Form von Tabletten verpreßbares Produkt ergibt.

Weiterhin hat es sich, wie bereits weiter oben angedeutet, als vorteilhaft erwiesen, wenn bereits aus dem Verfahren der Erfindung resultierende Thioctsäureware erneut als Vorlage dienen kann. Hierbei handelt es sich also denn um eine typische Aufbaugranulation.

Das Ergebnis des Aufsprühens einer Thioctsäurelösung auf eine Vorlage aus trockener oder wahlweise auch lösungsmittelfeuchter Thioctsäure im Wirbelbett eines geeigneten Wirbelschicht-Trockners läßt sich durch gezielte Einstellung einer Reihe von Parametern beeinflussen. So spielen für die Erfindung Parameter wie Produkttemperatur in der Wirbelschicht, Sprühdruck und Sprührate, Konzentration der aufzusprühenden Lösung sowie Art und Eigenschaft des verwendeten Lösungsmittels eine Rolle.

In besonders bevorzugter erfindungsgemäßer Verfahrensabwandlung wird eine hochkonzentrierte Lösung von Thioctsäure unter gezielter Einstellung von Produkttemperatur, Sprühdruck und Sprührate auf die Vorlage aufgesprüht, so daß das Lösungsmittel verdampft und Thioctsäure auskristallisiert.

Dabei kann die Wirbelschicht, also das Produkt im Wirbelbett, grundsätzlich bei jeder für die Thioctsäure verträglichen Temperatur gehalten werden. In bevorzugter Ausführungsform des erfindungsgemäßen Verfahrens wird daher die Sprühlösung in ein Produkt-Wirbelbett von 0 bis 60 °C eingesprüht. Besonders bevorzugt ist ein Temperaturbereich von 20 bis 40 °C für das Produkt-Wirbelbett.

Hinsichtlich des Sprühdruckes ist das Verfahren an sich nicht besonders kritisch. Das erfindungsgemäße Verfahren kann sowohl unter Normaldruck, leichtem Überdruck als auch im Vakuum durchgeführt werden. Das Verfahren der Erfindung kennzeichnet sich daher dadurch, daß das Versprühen bei 100 bis 1200 mbar durchgeführt wird. Besonders bevorzugte Druckbereiche liegen unterhalb des Normaldrucks bei einem leichten Vakuum von ca. 600 bis 800 mbar. In jedem Fall wird es für die Erfindung besonders vorteilhaft sein, wenn das Versprühen so durchgeführt wird, daß es in der Wirbelschicht zu einer Sprühkristallisation kommt. Damit ist gemeint, daß das Produkt in der Wirbelschicht während des erfindungsgemäßen Verfahrens in eine eher kristalline Form übergeht.

Die Sprührate selbst, d. h also das Gewicht der Lösung in Gramm, welche in der Zeiteinheit gemessen in Minuten Auf die Vorlage im Wirbelbett aufgesprüht wird, richtet sich nach verschiedenen Kriterien. So sind u. a. zu berücksichtigen: Die Größe resp. das Volumen der Vorrichtung, die das Wirbelbett umfaßt; das Fortschreiten der Produktbildung, denn eine zunehmende Produktmenge im Wirbelbett läßt eine Steigerung der Sprührate zu; schließlich steuert und beeinflußt die Sprührate die Korngröße selbst und die Korngrößenverteilung; generell führt eine größere Sprührate zu einem größeren Korn.

Als Lösungsmittel für die aufzusprühende Lösung von Thioctsäure kommen eine große Reihe von Verbindungen in Frage. So soll das Lösungsmittel die Bedingung erfüllen, daß Restmengen des Lösungsmittels im Verfahrensprodukt physiologisch tolerierbar sein sollen, und insbesondere muß die Restmenge des Lösungsmittels mit einfachen Mitteln auf die gesetzlichen Grenzwerte oder darunter absenkbar sein. Ferner soll das Lösungsmittel über die Eigenschaft verfügen eine möglichst große Menge an Thioctsäure aufzunehmen und zu lösen. Schließlich soll das Lösungsmittel inert gegen das Produkt sein, eine möglichst niedrige Verdampfungsenthalpie aufweisen und diese Eigenschaften vor allem im für die Thioctsäure verträglichen Temperaturbereich besitzen. Als Lösungsmittel können für die Erfindung beispielsweise dienen aliphatische Kohlenwasserstoffe mit einer Kohlenstoff-Kettenlänge zwischen 3 und 10 Kohlenstoffatomen, cycloaliphatische Kohlenwasserstoffe wie Cyclopentan, Cyclohexan, Methylcyclohexan, aromatische Kohlenwasserstoffe die flüssig sind, aliphatische oder cycloaliphatische Alkohole mit 2 bis 6 Kohlenstoffatomen, Ester aus aliphatischen Carbonsäuren mit 2 bis 6 Kohlenstoffatomen oder cycloaliphatischen Carbonsäuren mit 3 bis 6 Kohlenstoffatomen und aliphatischen Alkoholen mit 2 bis 6 Kohlenstoffatomen oder cycloaliphatischen Alkoholen mit 3 bis 6 Kohlenstoffatomen, aliphatische oder cycloaliphatische Ketone mit 3 bis 10 Kohlenstoffatomen, Ether und Glycolether oder homogene Mischungen der genannten Lösungsmittel. Besonders bevorzugte Lösungsmittel, die vor allem den oben angegebenen Anforderungen hervorragend entsprechen, sind u. a. Aceton und/oder Essigester.

Die Konzentration der Sprühlösung kann erfindungsgemäß über einen großen Bereich differieren. So liegt die Konzentration der Sprühlösung bei einer Verfahrensvariante der Erfindung zwischen ca. 10 und 80 %. Erfindungsgemäß werden möglichst hohe Bereiche angestrebt, da hierdurch vorteilhaft der Aufwand und die benötigte Lösungsmittelmenge für die Sprühlösung deutlich reduziert werden können.

Gemäß dem Verfahren der Erfindung resultiert in bevorzugter Ausführungsform ein rieselfähiges Produkt mit einem Schüttgewicht zwischen 500 und 900 g/l, das für eine galenische Weiterverarbeitung für feste Darreichungsformen und insbesondere für die Verpressung zu hochkonzentrierten festen Darreichungsformen hervorragend geeignet ist, insbesondere eine geeignete Korngrößenverteilung und Partikelbeschaffenheit aufweist. Darüber hinaus ist bei der erfindungsgemäßen Ware der Gehalt an Restlösungsmittel im Vergleich zu Thioctsäure, beispielsweise gemäß dem Verfahren wie es aus dem Stand der Technik in Form der DE-A 42 35 912 bekannt ist, deutlich niedriger. Daher zeichnet sich das Verfahren gemäß der Erfindung in einer bevorzugten Variante dadurch aus, daß ein Produkt mit einem Restlösungsmittelgehalt an organischen Lösungsmitteln von insgesamt < 1000 ppm erhalten wird.

Gegenstand der Erfindung ist auch eine neue feste, trockene R,S-Thioctsäure in Form eines Aufbaugranulates, die durch eine spezifische Oberfläche von > 0,7 m²/g und einen Anteil an Mesoporen mit einem Durchmesser zwischen 2 und 30 nm gekennzeichnet ist.

Die spezifische Oberfläche der erfindungsgemäßen R,S-Thioctsäure läßt sich nach dynamischen Untersuchungsverfahren, die dem Fachmann bekannt sind, bestimmen.

Gegenüber bekannten R,S-Thioctsäuren weist die erfindungsgemäße Ware eine deutlich größere spezifische Oberfläche auf. Bevorzugt liegt die spezifische Oberfläche der R,S-Thioctsäure gemäß der Erfindung im Bereich von 0,8 - 1,2 m²/g, besonders bevorzugt zwischen 0,84 und 1,05 m²/g.

Darüber hinaus verfügt die erfindungsgemäße R,S-Thioctsäure über einen Anteil sogenannter Mesoporen. Dies sind Poren mit einem Durchmesser zwischen 2 - 30 nm, die bei herkömmlichen Thioctsäuren nicht nachweisbar sind. Mikroporen mit einem Durchmesser von < 2 nm sind bei der erfindungsgemäßen R,S-Thioctsäure nicht nachweisbar.

In bevorzugter Ausführungsform ist die R,S-Thioctsäure der Erfindung dadurch gekennzeichnet, daß das Volumen der Mesoporen mit einem Durchmesser zwischen 2 und 30 nm pro Gramm R,S-Thioctsäure zwischen 0,001 und 0,005 ml/g ist.

Die Porenvolumina wurden dabei nach DIN 66 133 (geändert ab 01.10.92) bestimmt.

Neben den Mesoporen weisen die Thioctsäuren der Erfindung auch sogenannte Makroporen auf. Hierbei handelt es sich um solche mit einem Durchmesser von > 30 nm.

Deren Porenvolumen pro g Substanz ist i. d. R. sehr viel größer als das der Mesoporen.

Besonders günstig ist es, wenn das Verhältnis des Volumens von Mesoporen mit einem Durchmesser zwischen 2 und 30 nm pro Gramm R,S-Thioctsäure zum Volumen von Makroporen mit einem Durchmesser > 30 pro Gramm R,S-Thioctsäure zwischen 1 : 1000 und 1 : 10, bevorzugt zwischen 1 : 200 und 1 : 50, ist.

Wie ausgeführt, kann die Korngrößenverteilung des erfindungsgemäßen Produktes gezielt durch Einstellung der Parameter, wie Produkttemperatur, Sprühdruck, Sprührate, Beschaffenheit der Sprühdüse und durch variieren der Sprühdüsenposition, beeinflußt werden. Bei der Weiterverarbeitung der erfindungsgemäßen Ware zu Tabletten werden die bei den bisher erhältlichen Thioctsäure-Formen zu beobachtenden Eigenschaften, wie Anhaften am Preßwerkzeug oder Neigung zu Rißbildungen an der Tablette, nicht beobachtet. Daher kann auf ein besonders aufwendiges Granulationsverfahren bei der galenischen Weiterverarbeitung verzichtet werden.

Nachfolgend wird die Erfindung anhand von Beispielen weitergehend erläutert.

### Beispiel 1

Wirbelschichttrocknungsanlage Fa. Glatt GPCG 1 / Topspray mit Zuluft/Abluft; Zweistoffdüse d = 1,2 mm; Düseneinhau "oben,"; Sprühdruck: 1,4 bar

| | |
|---|---|
| Produktvorlage | 250 g Thioctsäure (gesiebt < 1250 µ) |
| Sprühlösung | 750 g Thioctsäure, 1125 g Essigester |

Die Produktvorlage wird mit Hilfe der ca. 40 °C warmen Zuluft auf 30 °C aufgeheizt und bei dieser Produkttemperatur mit dem Einsprühen der Lösung begonnen. Sprührate: 25 g/min., wobei die Dosierung allmählich auf 58 g/min. erhöht wird. Die Zulufttemperatur wird konstant bei 40 °C gehalten.
Die Produkttemperatur stellt sich auf 28 - 32 °C ein.

Nach beendeter Einsprühung läßt man 10 min. ebenfalls bei einer Produkttemperatur von 30 °C nachtrocknen.

### Ergebnis:

| | |
|---|---|
| Ausbeute | 890 g rieselfähiges Produkt |
| Siebanalyse | 94 % < 500 µ |
| Schüttgewicht | 616 g/l |

### Beispiel 2

Wirbelschichttrocknungsanlage Fa. Glatt GPCG 1 / Topspray mit Zweistoffdüse d = 1,2 mm; Düseneinbau "oben"; Sprühdruck: 1,4 bar

| | |
|---|---|
| Produktvorlage | 250 g Thioctsäure (gesiebt < 1250 µ) |
| Sprühlösung | 750 g Thioctsäure, 1125 g Aceton |

Die Produktvorlage wird mit Hilfe der ca. 30 °C warmen Zuluft auf 20 °C aufgeheizt und bei dieser Produkttemperatur mit dem Einsprühen der Lösung begonnen. Sprührate: 38 g/min., wobei die Dosierung allmählich auf 61 g/min. erhöht wird. Die Zulufttemperatur wird konstant bei 30 °C gehalten.
Die Produkttemperatur stellt sich auf 19 - 22 °C ein.

Nach beendeter Einsprühung läßt man 10 min. bei einer 20 Produkttemperatur von 30 °C nachtrocknen.

### Ergebnis:

| | |
|---|---|
| Ausbeute | 910 g rieselfähiges Produkt |
| Siebanalyse | 99 % < 500 µ |
| Schüttgewicht | 680 g/l |

### Beispiel 3

Wirbelschichttrocknungsanlage Fa. Glatt GPCG 1 / Topspray mit Zweistoffdüse d = 1,2 mm; Düseneinbau "oben"; Sprühdruck: 1,4 bar

| | |
|---|---|
| Produktvorlage | 250 g Thioctsäure (gesiebt < 1250 µ) |
| Sprühlösung | 750 g Thioctsäure, 1125 g Aceton |

Die Produktvorlage wird mit Hilfe der ca. 40 °C warmen Zuluft auf 30 °C aufgeheizt und bei dieser Produkttemperatur mit dem Einsprühen der Lösung begonnen. Sprührate: 25 g/min., wobei die Dosierung allmählich auf 59 g/min. erhöht wird. Die Zulufttemperatur wird konstant bei 40 °C gehalten.
Die Produkttemperatur stellt sich auf 28 - 32 °C ein.

Nach beendeter Einsprühung läßt man 10 min. bei einer Produkttemperatur von 30 °C nachtrocknen.

### Ergebnis:

| | |
|---|---|
| Ausbeute | 970 g rieselfähiges Produkt |
| Siebanalyse | 99 % < 500 µ |
| Schüttgewicht | 690 g/l |

### Beispiel 4

Wirbelschichttrocknungsanlage Fa. Glatt GPCG 1 / Topspray mit Zweistoffdüse d = 1,2 mm; Düseneinbau "oben"; Sprühdruck:. 1,4 bar

| | |
|---|---|
| Produktvorlage | 250 g Thioctsäure (gesiebt < 1250 µ) |
| Sprühlösung | 750 g Thioctsäure, 1125 g Essigester |

Die Produktvorlage wird mit Hilfe der ca. 30 °C warmen Zuluft auf 20 °C aufgeheizt und bei dieser Produkttemperatur mit dem Einsprühen der Lösung begonnen. Sprührate: 40 g/min., wobei die Dosierung allmählich auf 65 g/min. erhöht wird. Die Zulufttemperatur wird konstant bei 30 °C gehalten.
Die Produkttemperatur stellt sich auf 19 - 23 °C ein.

Nach beendeter Einsprühung läßt man 10 min. bei einer Produkttemperatur von 30 °C nachtrocknen.

### Ergebnis:

| | |
|---|---|
| Ausbeute | 920 g rieselfähiges Produkt |
| Siebanalyse | 99 % < 500 µ |
| Schüttgewicht | 686 g/l |

### Beispiel 5

Wirbelschichttrocknungsanlage Fa. Glatt GPCG 1 / Topspray mit Zweistoffdüse d = 1,2 mm; Düseneinbau "oben"; Sprühdruck: 1,4 bar

| | |
|---|---|
| Produktvorlage | 250 g Thioctsäure (gesiebt < 1250 µ) |
| Sprühlösung | 750 g Thioctsäure, 1125 g Ethanol |

Die Produktvorlage wird mit Hilfe der ca. 30 °C warmen Zuluft auf 20 °C aufgeheizt und bei dieser Produkttemperatur mit dem Einsprühen der Lösung begonnen. Sprührate: 43 g/min., wobei die Dosierung allmählich auf 64 g/min. erhöht wird. Die Zulufttemperatur wird konstant bei 30 °C gehalten.
Die Produkttemperatur stellt sich auf 20 - 23 °C ein.

Nach beendeter Einsprühung läßt man 10 min. bei einer Produkttemperatur von 30 °C nachtrocknen.

### Ergebnis:

| | |
|---|---|
| Ausbeute | 912 g rieselfähiges Produkt |
| Siebanalyse | 98 % < 500 µ |
| Schüttgewicht | 680 g/l |

Für die Beispiele 6 und 7 wurde trockene Thioctsäure mit folgenden Gehalten an Restlösungsmitteln eingesetzt:

| | |
|---|---|
| Cyclohexan | 3200 ppm |
| Essigester | 1900 ppm |

### Beispiel 6

Wirbelschichttrocknungsanlage Fa. Glatt GPCG 15 / Topspray mit Kreisgasführung; Zweistoffdüse d = 2,2 mm; Düseneinbau "oben"; Sprühdruck: 1,8 bar

| | |
|---|---|
| Produktvorlage | 4 kg Thioctsäure (granulierte Ware) |
| Sprühlösung: | 16,8 kg Thioctsäure, 25 kg Aceton |

Luftmenge (Wirbelschicht): 400 - 550 m³/h

Die Produktvorlage wird mit Hilfe der ca. 30 °C warmen Zuluft auf 20 °C aufgeheizt und bei dieser Produkttemperatur mit dem Einsprühen der Lösung begonnen. Sprührate: 330 g/min., wobei die Dosierung allmählich auf 620 g/min. erhöht wird. Die Zulufttemperatur wird konstant bei 30 °C gehalten. -
Die Produkttemperatur stellt sich auf 16 - 18 °C ein.

Nach beendeter Einsprühung läßt man 10 min. bei einer Produkttemperatur von 30 °C nachtrocknen.

### Ergebnis:

| | | |
|---|---|---|
| Ausbeute | 19,9 kg rieselfähiges Produkt | |
| Gehalt | 98,3 Gew.% | |
| Polymergehalt | < 2 Gew.% | |
| Siebanalyse | 1 % | > 1000 µ |
| | 11 % | > 710 µ |
| | 70 % | > 500 µ |
| | 9 % | > 355 µ |
| | 8 % | > 250 µ |
| | 1 % | > 100 µ |
| Schüttgewicht | 770 g/l | |
| Gehalt an Restlösungsmittel | Cyclohexan: | 104 ppm |
| | Essigester: | 22 ppm |
| | Aceton: | 345 ppm |

### Beispiel 7

Wirbelschichttrocknungsanlage Fa. Glatt GPCG 15 / Topspray mit Kreisgasführung; Zweistoffdüse d = 2,2 mm; Düseneinbau "Mitte"; Sprühdruck 1,2 -1,5 bar

| | |
|---|---|
| Produktvorlage | 4 kg Thioctsäure (gesiebte Ware) |
| Sprühlösung | 16,8 kg Thioctsäure, 25 kg Aceton |

Luftmenge (Wirbelschicht): 390 - 600 m³/h

Die Produktvorlage wird mit Hilfe der ca. 30 °C warmen Zuluft auf 20 °C aufgeheizt und bei dieser Produkttemperatur mit dem Einsprühen der Lösung begonnen. Sprührate: 386 g/min., wobei die Dosierung allmählich auf 470 g/min. erhöht wird. Die Zulufttemperatur wird konstant bei 30 °C gehalten.
Die Produkttemperatur stellt sich auf 19 - 20 °C ein.

Nach beendeter Einsprühung läßt man 10 min. bei einer Produkttemperatur von 30 °C nachtrocknen.

### Ergebnis:

| | | |
|---|---|---|
| Ausbeute | 19,3 kg rieselfähiges Produkt | |
| Gehalt | 98,5 Gew.% | |
| Polymergehalt | < 2 Gew.% | |
| Siebanalyse | 3 % | > 1000 µ |
| | 5,1 % | > 710 µ |
| | 53 % | > 500 µ |
| | 37,5 % | > 250 µ |
| | 0,5 % | > 100 µ |
| Schüttgewicht | 728 g/l | |
| Gehalt an Restlösungsmittel | Cyclohexan: | 576 ppm |
| | Essigester: | 151 ppm |
| | Aceton: | 257 ppm |

### Beispiel 8

Wirbelschichttrocknungsanlage Fa. Glatt GFCG 15 / Topspray mit Kreisgasführung; Zweistoffdüse d = 2,2 mm; Düseneinbau "Mitte"; Sprühdruck: 2 bar

| | |
|---|---|
| Produktvorlage | 4 kg Thioctsäure (feuchte Ware) |
| Sprühlösung | 10 kg Thioctsäure, 18 kg Essigester |

Luftmenge (Wirbelschicht): 500 m³/h

Die Produktvorlage wird mit Hilfe der ca. 30 °C warmen Zuluft auf 26 °C aufgeheizt und 10 min. bei dieser Produkttemperatur vorgetrocknet. Anschließend wird mit dem Einsprühen der Lösung begonnen. Sprührate: 240 g/min, wobei die Dosierung allmählich auf 500 g/min. erhöht wird. Die Zulufttemperatur wird konstant bei 30 °C gehalten.
Die Produkttemperatur stellt sich.auf.19 - 24 °C ein.

Nach beendeter Einsprühung läßt man 10 min. bei einer Produkttemperatur von 35 °C nachtrocknen.

### Ergebnis:

| | | |
|---|---|---|
| Ausbeute | 11,5 g rieselfähiges Produkt | |
| Gehalt | 99,6 Gew.% | |
| Polymergehalt | < 1 Gew.% | |
| Siebanalyse | 0,10 % | > 2000 µ |
| | 0,40 % | > 1400 µ |
| | 0,90 % | > 1000 µ |
| | 2,19 % | > 710 µ |
| | 4,78 % | > 500 µ |
| | 6,39 % | > 355 µ |
| | 33,3 % | > 250 µ |
| | 45,56 % | > 180 µ |
| | 6,18 % | > 125 µ |
| | 0,20 % | > 90 µ |
| | 0,00 % | > 63 µ |

### Beispiel 9

Zur weiteren Spezifikation und Beschreibung der erfindungsgemäßen Ware, wurden Untersuchungen zur Ermittlung der spez. Oberfläche und Porenverteilung durchgeführt.

Folgende Thioctsäure-Waren wurden untersucht:
Probe 1 (am Markt käufliches Muster)
Probe 2 (am Markt käufliches Muster)
Probe 3 (Einsatzprodukt für Beispiel 6 - 8)
Probe 4 (erfindungsgemäße Ware resultierend aus Beispiel 6)
Probe 5 (erfindungsgemäße Ware resultierend aus Beispiel 7)
Probe 6 (erfindungsgemäße Ware resultierend aus Beispiel 8)
Folgende Bestimmungen wurden durchgeführt:
   1. Bestimmung der spezifischen Oberfläche nach dem Trägergasverfahren (Einpunktmethode): Die Methode ist prinzipiell (ohne nähere Verfahrensparameter) in DIN 66131, Abs. 4.3.4 beschrieben.
      In ASTM D 4567-86 ("Standard Test Method for Single Point Determination of Specific Surface Area of Catalysts Using Nitrogen Adsorption by Continuous Flow Method") ist diese Vorschrift ausführlich beschrieben. Abweichend von dieser Vorschrift wurde die Entgasung im Stickstoffstrom bei Raumtemperatur durchgeführt.
   2. Die Bestimmung des Mesoporenvolumens und der -verteilung erfolgte durch die Gerätesoftware des ASAP 2400 (Fa. Micromeritics) aus der statisch-volumetrisch gemessenen Sorptionsisotherme von Stickstoff bei T = 77 K (vergl. DIN 66131): Das Mesopoenlvolumen wird durch Umrechnung des bei p/pₒ = 0,931 (entspricht einem Porendurchmesser von ca. 30 nm) adsorbierten Gasvolumens auf Kondensatvolumen ggf. unter Berücksichtigung des Mikroporenvolumens erhalten. Die Mesoporenverteilung wird nach der Methode von Barett, Joyner und Halenda (ASTM D 4641-88) aus der Sorptionsisotherme berechnet. Dieses Verfahren ist in der DIN 66134 (8. Normvorlage) beschrieben. Die Probenvorbehandlung erfolgte bei Raumtemperatur unter Vakuum.

### Ergebnis

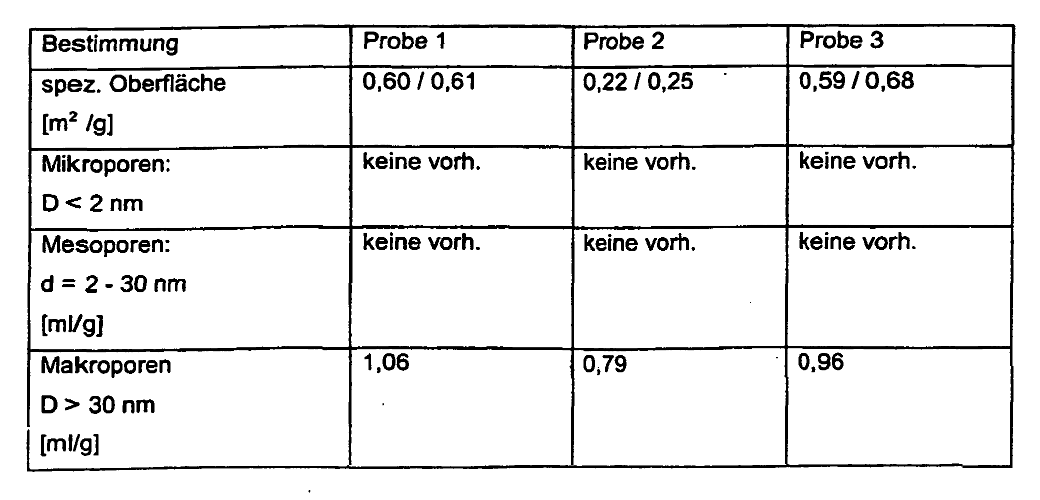

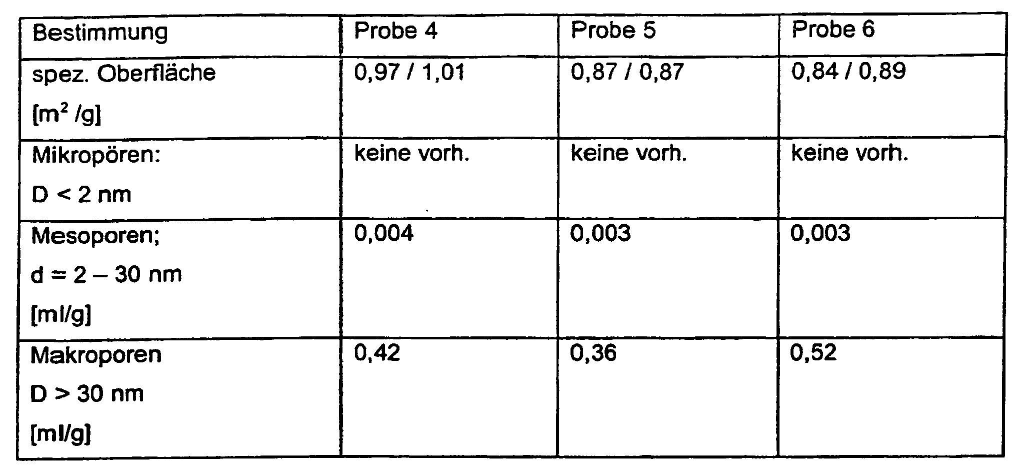

Im Makroporenbereich wurden hier überwiegend Kornzwischenräume gemessen.

## Patentansprüche

1. Verfahren zur Darstellung von rieselfähiger Thioctsäure, die durch Verpressung zu festen Darreichungsformen mit einem Wirkstoffanteil > 80% und Wirkstoffgehalten von mehr als 200 mg Reinsubstanz galenisch weiterverarbeitet werden kann,
**dadurch gekennzeichnet, daß** mehr als 10 Gew. % und weniger als 50 Gew. % Thioctsäure beliebigen Ursprungs bezogen auf die Gesamtmenge an erhaltenem Produkt im Wirbelbett einer zur Erzeugung einer Wirbelschicht geeigneten Vorrichtung vorgelegt wird und daß durch Versprühen einer Lösung von Thioctsäure beliebigen Ursprungs auf die Vorlage unter gleichzeitiger Entfernung des Lösungsmittels ein aus Thioctsäure bestehendes Aufbaugranulat erhalten wird.

2. Verfahren nach Anspruch 1 **dadurch gekennzeichnet, daß** als stationäre Phase in der Wirbelschicht lösungsmittelfeuchte Thioctsäure eingesetzt wird.

3. Verfahren nach Anspruch 1 oder 2, **dadurch gekennzeichnet, daß** mehr als 10 Gew. % und weniger als 50 Gew. % Thioctsäure als Produktvorlage im Wirbelbett vorgelegt werden, bezogen auf 100 Gew. % trockene Thioctsäure umfassend die Summe aus vorgelegter und aufgesprühter Thioctsäure.

4. Verfahren nach Anspruch 3, **dadurch gekennzeichnet, daß** ca. 25 Gew. % Thioctsäure vorgelegt werden, bezogen auf 100 Gew. % trockene Thioctsäure umfassend die Summe aus vorgelegter und aufgesprühter Thioctsäure

5. Verfahren nach einem der Ansprüche 2 bis 4, **dadurch gekennzeichnet, daß** die lösungsmittelfeuchte Thioctsäure einem Herstellungsverfahren entstammt, bei dem man ein Teil Thioctsäure bei 10 °C bis 60 °C in 5 - 20 Teilen Lösungsmittel oder Lösungsmittelgemisch löst und innerhalb von 2 - 10 Stunden auf 0 °C bis -20 °C abkühlt, wobei das Lösungsmittel oder Lösungsmittelgemisch eine Dielektrizitätskonstante ε zwischen 2,5 und 5,5 aufweist,

6. Verfahren nach einem der Ansprüche 1,3 und 4, **dadurch gekennzeichnet, daß** die im Wirbelbett vorzulegende Thioctsäure dem Verfahren gemäß Anspruch 1 entstammt.

7. Verfahren nach einem der vorhergehenden Ansprüche, **dadurch gekennzeichnet, daß** eine Lösung von Thioctsäure mit einer Konzentration zwischen 10 und 80% unter gezielter Einstellung von Produkttemperatur, Sprühdruck und Sprührate auf die Vorlage aufgesprüht wird, so daß das Lösungsmittel verdampft und Thioctsäure auskristallisiert.

8. Verfahren nach Anspruch 7, **dadurch gekennzeichnet, daß** die Sprühlösung in ein Produktwirbelbett mit einer Temperatur im Bereich von 0 bis 60 °C eingesprüht wird.

9. Verfahren nach Anspruch 8, **dadurch gekennzeichnet, daß** die Sprühlösung in ein Produktwirbelbett mit einer Temperatur im Bereich von 20 bis 40 °C eingesprüht wird.

10. Verfahren nach Anspruch 7 oder 8, **dadurch gekennzeichnet, daß** das Aufsprühen bei 100 bis 1200 mbar durchgeführt wird.

11. Verfahren nach Anspruch 10, **dadurch gekennzeichnet, daß** das Aufsprühen bei 600 bis 800 mbar durchgeführt wird.

12. Verfahren nach einem der vorhergehenden Ansprüche, **dadurch gekennzeichnet, daß** aromatische, aliphatische oder cycloaliphatische organische Lösungsmittel zur Herstellung der Sprühlösung verwendet werden, bevorzugt Aceton und/oder Essigester.

13. Verfahren nach einem der vorhergehenden Ansprüche, **dadurch gekennzeichnet, daß** ein rieselfähiges Produkt mit einem Schüttgewicht zwischen 500 und 900 g/l erhalten wird, wobei im Sinne einer Sprühtrocknung gleichzeitig das Lösungsmittel entfernt und die Thioctsäure auf die Vorlage ähnlich einer Aufbaugranulation oder ähnlich eines Wirbelschichtcoatingverfahrens aufgebracht wird, das eine für die galenische Weiterverarbeitung für feste Darreichungsformen und insbesondere für die Verpressung zu hochkonzentrierten festen Darreichungsformen geeignete Korngrößenverteilung und Partikelbeschaffenheit aufweist.

14. Verfahren nach einem der vorhergehenden Ansprüche, **dadurch gekennzeichnet, daß** ein Produkt mit einem Restlösungsmittelgehalt an organischen Lösungsmitteln von insgesamt < 1000 ppm erhalten wird, wobei im Sinne einer Sprühtrocknung gleichzeitig das Lösungsmittel entfernt und die Thioctsäure auf die Vorlage ähnlich einer Aufbaugranulation oder ähnlich eines Wirbelschichtcoatingverfahrens aufgebracht wird.

15. Feste, trockene R,S-Thioctsäure in Form eines Aufbaugranulates, **gekennzeichnet durch** eine spezifische Oberfläche von > 0,7 m²/g und einen Anteil an Mesoporen mit einem Durchmesser zwischen 2 und 30 nm.

16. Feste, trockene R,S-Thioctsäure in Form eines Aufbaugranulates nach Anspruch 15, **dadurch gekennzeichnet, daß** das Volumen der Mesoporen mit einem Durchmesser zwischen 2 und 30 nm pro Gramm R,S-Thioctsäure zwischen 0,001 und 0,005 mUg ist.

17. Feste, trockene R,S-Thioctsäure in Form eines Aufbaugranulates nach Anspruch 15 oder 16, **dadurch gekennzeichnet, daß** das Verhältnis des Volumens von Mesoporen mit einem Durchmesser zwischen 2 und 30 nm pro Gramm R,S-Thioctsäure zum Volumen von Makroporen mit einem Durchmesser > 30 nm Pro Gramm R,S-Thioctsäure zwischen 1 : 1000 und 1 : 10, bevorzugt zwischen 1 : 200 und 1 : 50, ist.

18. Verwendung von Thioctsäure nach einem oder mehreren der vorstehenden Ansprüche zur Herstellung von Arzneimitteln.

## Claims

1. Process for the preparation of free-flowing thioctic acid which can be further pharmaceutically processed by compression to solid dosage forms having a proportion of > 80% active ingredient and active ingredient content of more than 200 mg of pure substance, **characterized in that** more than 10% by weight and less than 50% by weight of thioctic acid of any origin, based on the total amount of product obtained, is introduced into the fluidized bed of an apparatus suitable for generating a fluidized bed, and **in that** agglomerated granules consisting of thioctic acid are obtained by spraying a solution of thioctic acid of any origin onto the introduced material with simultaneous removal of the solvent.

2. Process according to Claim 1, **characterized in that** solvent-moist thioctic acid is employed as stationary phase in the fluidized bed.

3. Process according to Claim 1 or 2, **characterized in that** more than 10% by weight and less than 50% by weight of thioctic acid are introduced as introduced product into the fluidized bed, based on 100% by weight of dry thioctic acid comprising the total of introduced and sprayed-on thioctic acid.

4. Process according to Claim 3, **characterized in that** about 25% by weight of thioctic acid are introduced, based on 100% by weight of dry thioctic acid comprising the total of introduced and sprayed-on thioctic acid.

5. Process according to any of Claims 2 to 4,
**characterized in that** the solvent-moist thioctic acid is derived from a preparation process in which one part of the thioctic acid is dissolved in 5-20 parts of solvent or solvent mixture at 10°C to 60°C, and cooled over the course of 2-10 hours to 0°C to -20°C, the solvent or solvent mixture having a dielectric constant ε of between 2.5 and 5.5.

6. Process according to any of Claims 1, 3 and 4, **characterized in that** the thioctic acid introduced into the fluidized bed is derived from the process according to Claim 1.

7. Process according to any of the preceding claims, **characterized in that** a solution of thioctic acid with a concentration between 10 and 80% is sprayed onto the introduced material, with targeted adjustment of product temperature, spraying pressure and spraying rate, so that the solvent evaporates and thioctic acid crystallizes out.

8. Process according to Claim 7, **characterized in that** the spray solution is sprayed into a product fluidized bed with a temperature in the range from 0 to 60°C.

9. Process according to Claim 8, **characterized in that** the spray solution is sprayed into a product fluidized bed with a temperature in the range from 20 to 40°C.

10. Process according to Claim 7 or 8, **characterized in that** the spraying on is carried out at from 100 to 1200 mbar.

11. Process according to Claim 10, **characterized in that** the spraying on is carried out at from 600 to 800 mbar.

12. Process according to any of the preceding claims, **characterized in that** aromatic, aliphatic or cycloaliphatic organic solvents are used to prepare the spray solution, preferably acetone and/or ethyl acetate.

13. Process according to any of the preceding claims, **characterized in that** a free-flowing product with a bulk density of between 500 and 900 g/l is obtained with, in the manner of a spray drying, simultaneous removal of the solvent and application of the thioctic acid to the introduced material similar to an agglomerating granulation or to a fluidized bed coating process, which has a particle size distribution and particle characteristics suitable for further pharmaceutical processing for solid dosage forms and in particular for compression to highly concentrated solid dosage forms.

14. Process according to any of the preceding claims, **characterized in that** a product having a residual solvent content of organic solvents totalling < 1000 ppm is obtained with, in the manner of a spray drying, simultaneous removal of the solvent and application of the thioctic acid to the introduced material similar to an agglomerating granulation or to a fluidized bed coating process.

15. Solid, dry R,S-thioctic acid in the form of agglomerated granules, **characterized by** a specific surface area of > 0.7 m²/g and a proportion of mesopores having a diameter of between 2 and 30 nm.

16. Solid, dry R,S-thioctic acid in the form of agglomerated granules according to Claim 15, **characterized in that** the volume of the mesopores having a diameter of between 2 and 30 nm per gram of R,S-thioctic acid is between 0.001 and 0.005 ml/g.

17. Solid, dry R,S-thioctic acid in the form of agglomerated granules according to Claim 15 or 16, **characterized in that** the ratio of the volume of mesopores having a diameter of between 2 and 30 nm per gram of R,S-thioctic acid to the volume of macropores having a diameter of > 30 nm per gram of R,S-thioctic acid is between 1:1000 and 1:10, preferably between 1:200 and 1:50.

18. Use of thioctic acid according to one or more of the preceding claims for producing medicaments.

## Revendications

1. Procédé pour la préparation d'acide thioctique apte à l'écoulement, qui peut être transformé en forme galénique par compression en formes pharmaceutiques solides présentant une proportion de substance active de > 80 % et à teneurs en substance active de plus de 200 mg de substance pure, **caractérisé en ce qu'**on dispose au préalable dans le lit fluidisé d'un dispositif approprié à la production d'une couche fluidisée plus de 10 % en poids et moins de 50 % en poids d'acide thioctique d'origine quelconque, par rapport à la quantité totale de produit obtenu et en que par pulvérisation d'une solution d'acide thioctique d'origine quelconque sur la charge initiale, avec élimination simultanée du solvant, on obtient un produit granulé par recouvrement, constitué d'acide thioctique.

2. Procédé selon la revendication 1, **caractérisé en ce qu'**on utilise en tant que phase stationnaire dans la couche fluidisée de l'acide thioctique imprégné de solvant.

3. Procédé selon la revendication 1 ou 2, **caractérisé en ce qu'**on dispose au préalable en tant que charge initiale de produit dans le lit fluidisé plus de 10 % en poids et moins de 50 % en poids d'acide thioctique, par rapport à 100 % en poids d'acide thioctique sec comprenant la somme de l'acide thioctique disposé au préalable et de l'acide thioctique pulvérisé.

4. Procédé selon la revendication 3, **caractérisé en ce qu'**on dispose au préalable environ 25 % en poids d'acide thioctique, par rapport à 100 % en poids d'acide thioctique sec comprenant la somme de l'acide thioctique disposé au préalable et de l'acide thioctique pulvérisé.

5. Procédé selon l'une quelconque des revendications 2 à 4, **caractérisé en ce que** l'acide thioctique imprégné de solvant provient d'un procédé de préparation dans lequel une partie d'acide thioctique est dissoute à une température de 10 à 60°C dans 5-20 parties de solvant ou mélange de solvants, puis refroidie à une température de 0 à 20°C en l'espace de 2 à 10 heures, le solvant ou mélange de solvants présentant une constante diélectrique ε comprise entre 2,5 et 5,5.

6. Procédé selon l'une quelconque des revendications 1, 3 et 4, **caractérisé en ce que** l'acide thioctique disposé au préalable dans le lit fluidisé provient du procédé selon la revendication 1.

7. Procédé selon l'une quelconque des revendications précédentes, **caractérisé en ce qu'**on pulvérise sur la charge initiale une solution d'acide thioctique ayant une concentration comprise entre 10 et 80 %, avec réglage dans un but précis de la température du produit, de la pression de pulvérisation et de la vitesse de pulvérisation, de sorte que le solvant s'évapore et l'acide thioctique cristallise.

8. Procédé selon la revendication 7, **caractérisé en ce que** la solution de pulvérisation est introduite par pulvérisation dans un lit fluidisé de produit à une température dans la plage de 0 à 60°C.

9. Procédé selon la revendication 8, **caractérisé en ce que** la solution de pulvérisation est introduite par pulvérisation dans un lit fluidisé de produit à une température dans la plage de 20 à 40°C.

10. Procédé selon la revendication 7 ou 8, **caractérisé en ce que** la pulvérisation est effectuée sous une pression de 100 à 1 200 bars.

11. Procédé selon la revendication 10, **caractérisé en ce que** la pulvérisation est effectuée sous une pression de 600 à 800 bars.

12. Procédé selon l'une quelconque des revendications précédentes, **caractérisé en ce que** pour la préparation de la solution de pulvérisation on utilise des solvants organiques aromatiques, aliphatiques ou cycloaliphatiques, de préférence l'acétone et/ou l'acétate d'éthyle.

13. Procédé selon l'une quelconque des revendications précédentes, **caractérisé en ce qu'**on obtient un produit apte à l'écoulement ayant une densité apparente comprise entre 500 et 900 g/l, en éliminant simultanément le solvant dans le sens d'un séchage par atomisation et l'acide thioctique étant appliqué sur la charge initiale comme dans une granulation par recouvrement ou comme dans un procédé de revêtement en lit fluidisé, qui présente une forme de particule et une distribution granulométriques appropriées à la transformation galénique pour formes pharmaceutiques solides et en particulier pour la compression en formes pharmaceutiques solides fortement concentrées.

14. Procédé selon l'une quelconque des revendications précédentes, **caractérisé en ce qu'**on obtient un produit ayant une teneur en solvants organiques résiduels de < 1 000 ppm au total, en éliminant simultanément le solvant dans le sens d'un séchage par atomisation et l'acide thioctique étant appliqué sur la charge initiale comme dans une granulation par recouvrement ou comme dans un procédé de revêtement en lit fluidisé.

15. Acide R,S-thioctique solide, sec, sous forme d'un produit granulé par recouvrement, **caractérisé par** une surface spécifique de > 0,7 m²/g et une teneur en mésopores ayant un diamètre compris entre 2 et 30 nm.

16. Acide R,S-thioctique solide, sec, sous forme d'un produit granulé par recouvrement, selon la revendication 15, **caractérisé en ce que** le volume des mésopores ayant un diamètre compris entre 2 et 30 nm, par gramme d'acide R,S-thioctique, est compris entre 0,001 et 0,005 ml/g.

17. Acide R,S-thioctique solide, sec, sous forme d'un produit granulé par recouvrement, selon la revendication 15 ou 16, **caractérisé en ce que** le rapport du volume des mésopores ayant un diamètre compris entre 2 et 30 nm par gramme d'acide R,S-thioctique au volume des macropores ayant un diamètre > 30 nm par gramme d'acide R,S-thioctique est compris entre 1:1000 et 1:10, de préférence entre 1:200 et 1:50.

18. Utilisation d'acide thioctique selon une ou plusieurs des revendications précédentes, pour la fabrication de médicaments.
